(19) **Europäisches Patentamt**
**European Patent Office**
**Office européen des brevets**

(11) **EP 1 480 551 B1**

(12) **EUROPEAN PATENT SPECIFICATION**

(45) Date of publication and mention
of the grant of the patent:
**19.04.2017  Bulletin 2017/16**

(51) Int Cl.:
***A61B 3/103*** (2006.01)

(21) Application number: **03709168.3**

(86) International application number:
**PCT/US2003/004921**

(22) Date of filing: **13.02.2003**

(87) International publication number:
**WO 2003/068059 (21.08.2003 Gazette 2003/34)**

(54) **APPARATUS FOR OBJECTIVE CHARACTERIZATION OF VISION BASED ON WAVEFRONT SENSING**

VORRICHTUNG ZUR OBJEKTIVEN BESTIMMUNG DES VISUS AUF GRUNDLAGE EINER WELLENFRONT-MESSUNG

APPAREIL PERMETTANT LA CARACTERISATION OBJECTIVE DE LA VISION REPOSANT SUR LA DETECTION DU FRONT D'ONDE

(84) Designated Contracting States:
**AT BE BG CH CY CZ DE DK EE ES FI FR GB GR HU IE IT LI LU MC NL PT SE SI SK TR**

(30) Priority: **13.02.2002  US 76218**

(43) Date of publication of application:
**01.12.2004  Bulletin 2004/49**

(73) Proprietor: **Essilor International (Compagnie Générale d'Optique)**
**94220 Charenton-le-Pont (FR)**

(72) Inventors:
• **LAI, Shui, T.**
**Encinitas, CA 92024 (US)**
• **DREHER, Andreas, W.**
**Escondido, CA 92029 (US)**

(74) Representative: **f & e patent**
**Fleischer, Engels & Partner mbB, Patentanwälte**
**Braunsberger Feld 29**
**51429 Bergisch Gladbach (DE)**

(56) References cited:
EP-A- 1 153 570      WO-A-00/10448
WO-A-01/58339      DE-A- 4 222 395
US-A- 5 929 970

• LIANG J ET AL: "OBJECTIVE MEASUREMENT OF WAVE ABERRATIONS OF THE HUMAN EYE WITH THE USE OF A HARTMANN-SHACK WAVE-FRONT SENSOR" JOURNAL OF THE OPTICAL SOCIETY OF AMERICA, AMERICAN INSTITUTE OF PHYSICS. NEW YORK, US, vol. 11, no. 7, July 1994 (1994-07), pages 1949-1957, XP000955413
• BARSKY BRIAN A ET AL: "Incorporating camera models, ocular models, and actual patient eye data for photo-realistic and vision-realistic rendering" THE FIFTH INTERNATIONAL CONFERENCE ON MATHEMATICAL METHODS FOR CURVES AND SURFACES, OSLO, 29 June 2000 (2000-06-29) - 4 July 2000 (2000-07-04), pages 1-10, XP002192222 Retrieved from the Internet: <URL:http://www.cs.berkeley.edu/optical/vr ender/papers/barsky-rendering.pdf> [retrieved on 2002-03-04]

**Description**

Background of the Invention

Field of the Invention

The present invention relates generally to an apparatus for determining a person's visual characteristics, and more particularly to apparatus for determining the refraction of the eye.

Description of the Related Art

Phoropters are apparatus used by optometrists to determine a patient's visual characteristics, so that proper eye diagnoses can be made and eyewear can be prescribed. In conventional phoropters, a patient looks through the phoropter, in which various test lenses are disposed, at a target eye chart, referred to as a "Snellen chart," while an optometrist moves the test corrective lenses into the patient's field of view. In some applications, the target may be positioned at a predetermined distance from the patient. The patient is then asked to verbally compare the quality of the perceived image as afforded by one lens versus the prior lens presented. The optometrist takes note of either an improvement or a deterioration in the patient's vision through such lenses. Systematically, the test progresses towards the "best" test lens entirely based on the patient's responses. The lens parameters are then used as the basis for a prescription for eyewear.

Unfortunately, as recognized herein, the patient can become fatigued during the process and/or misjudge the vision afforded by the various lenses. This can lead to the selection of a less than optimum prescription. Moreover, some patients, such as a very ill or a very young patient, might not be capable of articulating the quality of vision the various lenses afford the patient.

Objective methods of determining the patient's refraction errors have been proposed, but these other methods introduce further complications that are not present when using phoropters. In a retinoscopy method, for example, a streak of light is projected to a patient's retina, and the characteristics of the reflected light at the patient's corneal plane is analyzed to determine whether the patient is myopic, or hyperopic, and with or without astigmatism. However, the method does not provide sufficient accuracy for prescribing spectacle lenses. Consequently, its measurement results are typically used only as a starting point of a standard phoropter measurement.

Another objective measurement instrument for determining refractive errors is an autorefractor, which, owing to its speed of use, is more popular than retinoscopy. To use the autorefractor, a patient is asked to look inside an enclosed box that is part of the autorefractor. A target image is optically projected into patient's eye, and a series of lenses is automatically moved into position of the patient's line of sight to the target, to neutralize the patient's refractive errors (autorefraction). Unfortunately, the measurement outcome often differs from the patient's ideal prescription. Accordingly, like retinoscopy, autorefractor outcomes typically are used only as starting points for standard phoropter measurements.

Moreover, both retinoscopy and autorefraction fail to account for the accommodation effect of the patient, that is, for the propensity of a patient to alter his or her focus or sight to make the best of the vision test. An autorefractor measurement essentially is a snapshot of the patient's vision at a particular instant at which the autorefractor has identified a so-called neutralization point, and at this point if it happens that the patient focuses his vision for seeing an image at a distance other than what is intended, or if the patient is momentarily looking elsewhere other than the target, the output of the autorefractor is erroneous. Such deceptive focussing on the part of the patient can arise because the patient is conscious of the working distance inside the box, and when an image of an object presented to the patient which is modeled to be located at, e.g., twenty feet, the patient automatically focuses for an image at a much closer distance, knowing the actual size of the box. Examination results that include patient accommodation effects are inaccurate for prescribing spectacle lenses.

Another limitation of the autorefractor is that the examiner has no control over which lens is to be used in the test. The result is that repeated measurements are likely to provide different results for the same eye from the same patient, which results in laborious and time consuming tests and retests when using the device to finalize a prescription.

The article "Objective measurement of wave aberrations of the human eye with the use of a Hartmann-Shack wave-front sensor", by LIANG J. et al, published in JOURNAL OF THE OPTICAL SOCIETY OF AMERICA, vol. 11, no. 7, July 1994, pages 1949-1957, shows the features of the preamble of claim 1. EP 1 153 570 A1 also shows the features of the preamble of claim 1.

Thus, what is needed is an improved apparatus for measuring the optical properties of a person's eye.

Summary of the Invention

The invention concerns an apparatus for automatically determining the patient's quality of vision as defined in claim 1 and a computer readable medium having a program of instructions stored thereon for causing the electrical processor of the apparatus of claim 1 to execute method steps for identifying optical correction for a person's eye based on said wavefront measurements of light reflected from said person's eye as defined in claim 7.

The dependent claims concern preferred embodiments. Aspects, embodiments or examples of the present disclosure which do not fall under the appended claims are not part of the present invention.

The details of the present invention, both as to its structure and operation, can be understood in reference to the accompanying drawings, in which like reference numerals refer to like parts.

Brief Description of the Drawings

Figure 1 is a perspective view of an apparatus for characterizing a patient's vision, in one intended environment;
Figure 2 is a perspective view of the apparatus, showing the patient;
Figure 3 is a block diagram of the components of one preferred apparatus;
Figure 4 is a flow chart of the some preferred logic;
Figures 5-9 are exemplary non-limiting diagrams of quality of vision displays; and
Figure 10 is a flow chart showing an automatic refraction method described herein.

Detailed Description of the Preferred Embodiment

Although this invention will be described in terms of certain preferred embodiments, the scope of the invention is defined only by reference to the appended claims.

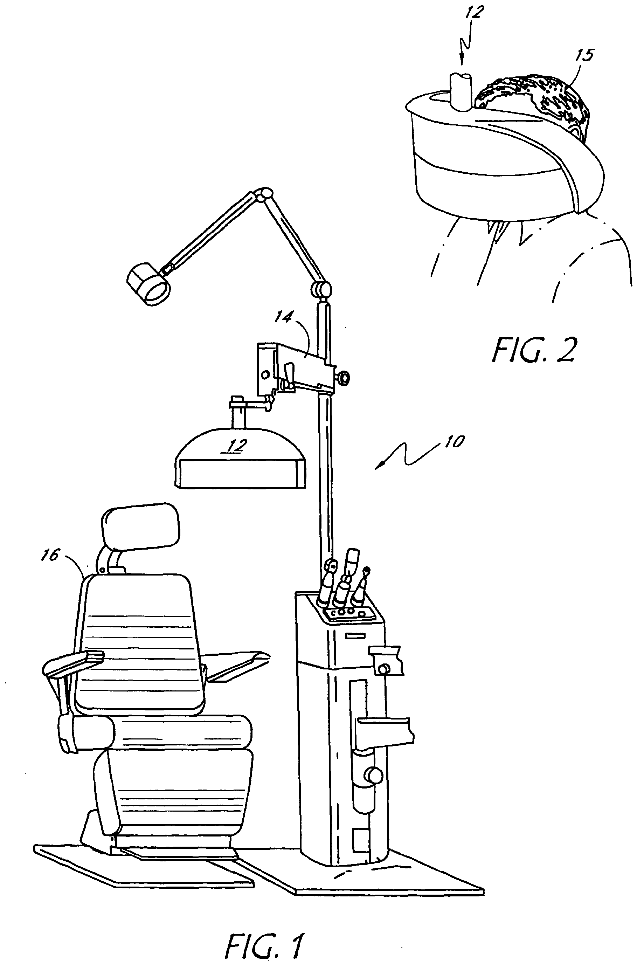

Referring initially to Figures 1 and 2, an exemplary apparatus is shown, generally designated 10, and includes a housing 12 that can be mounted on a movable stand 14 for positioning the housing 12 in front of a patient 15 who might sit in an examination chair 16. As can be appreciated in cross-reference to Figures 1 and 2, the patient 15 can position his or her head against the housing 12. Alternatively, the housing 12 can be supported on the head of the patient 15 and/or be suspended from a flexible overhanging arm which may be attached to a stand, to provide weight balance and to facilitate mounting and dismounting of the head mounted-apparatus. In other embodiments, the apparatus can be co-mounted with a conventional phoropter (not shown), in which case the test lenses, described more fully below, can be established by the lenses of the conventional phoropter. Other configurations may also be practicable.

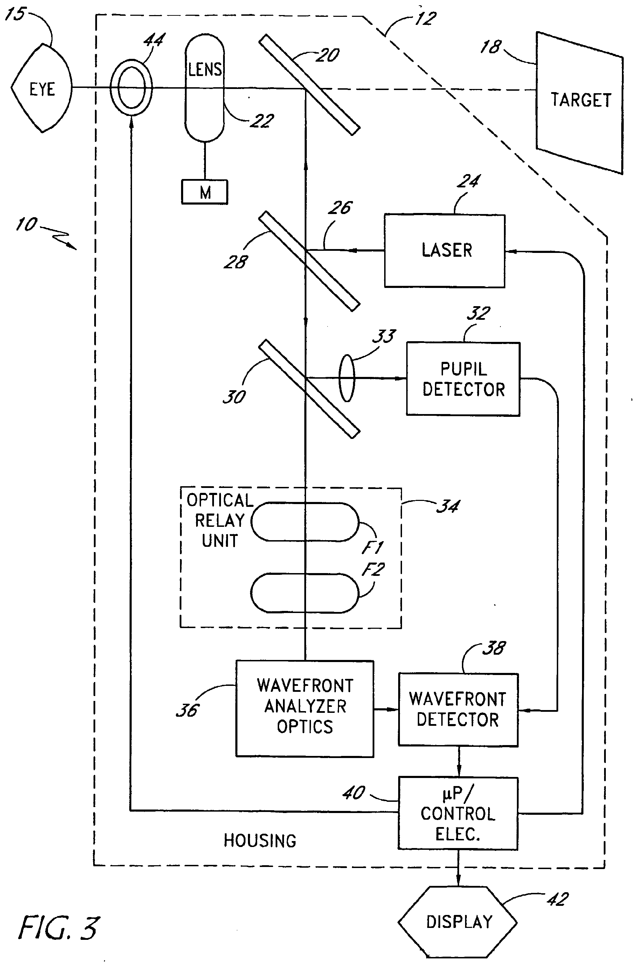

Now referring to Figure 3, the patient 15 can look through the housing 12 to a target 18, such as but not limited to a Snellen chart. The target 18 can be positioned at any appropriate distance from the patient 15, e.g., twenty feet or closer. Preferably, the target 18 is a distance $z_o$ of about 8 to 20 feet from the patient's eye 15. Since the target 18 can be positioned at a distance that actually is the distance intended, the above-noted patient accommodation effects related to autorefractors, are reduced or eliminated. In some cases, a mirror may be inserted in the optical path between the system 10 and the target 18 to introduce a turn in the optical path. These reflectors may be employed, for example, to accommodate suitable placement of the target 18 such as on the ceiling of an office where the vision tests are being administered.

Figure 3 shows one exemplary implementation of the housing 12. While Figure 3 shows that various components are located inside the housing 12 and various other components such as the output display are located outside the housing 12, it is to be understood that the principles advanced herein apply to systems having multiple housings, or a single housing. Additionally, other component and configurations may be included in the design.

In the embodiment shown, the patient looks at the target 18 through a transparent window within the housing 12, such as can be established by a primary beamsplitter 20 or other optical element. Interposed in the line of sight of the patient 15 are one or more movable test lens 22. By "movable" is meant physically movable by hand or computer or electronically-controlled mechanism as indicated as "M" in Figure 3, and more fully disclosed below, such that the lens is selectively interposed in or out of the line of sight of the patient 15. The test lens may also be movable in the sense that a variable focal length lens can be used, with its optical characteristics being variable in accordance with principles known in the art, for example those utilized in various designs of autorefractors. In certain embodiments, the test lens 22 can be, but should not be limited to, a single concave convex lens, or a combination of optical components such as lenses, and may include a cylindrical lens and a prism.

The test lens 22 may be disposed in an optical path between the primary beamsplitter 20 and the patient's eye 15 as depicted in Figure 3. The distance $y_o$ between the test lens 22 and the eye is relatively small, compared, for example, to the distance to the target 18. The separation between the eye 15 and the lens 22 may, e.g., be between about 0.8 and 1.6 centimeters in some embodiments. In the case, where this separation is small, the distance from the test lens 22 to the physical target, $z_o - y_o$, is approximately equal to the distance $z_o$ from the eye to the target 18.

As also shown, a light source such as but not limited to a laser 24 generates a light beam 26 that can be directed, in one preferred embodiment, toward a laser beamsplitter 28. The laser beamsplitter 28 reflects the light beam 26 toward the primary beamsplitter 20, which in turn reflects the beam through the test lens 22 and onto the eye of the patient.

Note that in certain embodiments, the test lens 22 may be inserted in the optical path between the beamsplitter 20 and the target such that the laser beam 26 does not pass therethrough. However, in either case where the test lens 22 is employed, preferably the vision is at least partially corrected. Measurements may be more accurate when vision is good and the eye is not stressed and is operating more normally.

With reference again to Figure 3, after reaching the eye, the beam 26 is reflected therefrom, back through the lens 22, and is reflected off the primary beamsplitter 20. The beam passes through the laser beamsplitter 28, and a portion of the beam is reflected off a pupil detection beamsplitter 30 toward a pupil light detector 32 through one or more focussing lenses 33, for purposes to be shortly disclosed. A portion of the return beam passes through the pupil detection beamsplitter 30 and propagates through an optical relay unit 34, which focuses the beam onto a wavefront analyzer optics 36. The wavefront analyzer optics 36 generates a signal representative of the wavefront of the return beam, and a wavefront detector 38 transforms the signal into an electrical signal for analysis by a processor 40. In one preferred embodiment, the processor 40 can be associated with control electronics known in the art for undertaking control of one or more components (e.g., the light source 24) of the system 10 as more fully set forth below. Also, the processor 40 can generate the below-described visual indications of the patient's vision as corrected by the test lens 22 and can cause the indications to be displayed on a display device 42, such as a video monitor, that can be mounted on the housing 12 or apart therefrom. The display device 42 can be a liquid crystal display that can be mounted on the housing 12 of the system 10, or a stand-alone display unit conveniently located for the examiner's viewing. Suitable displays may include, but not be limited to, numerical and/or graphical representations indicative of the patient's quality of vision, as described more fully below.

If desired, an illumination light 44, e.g., a ring-shaped fiber optic, can be mounted on the housing 12 and can be connected to the processor 40 to control the pupil size of the patient 15. The illumination light 44 can be a source of diffused light. The light intensity of the illumination light 44 may be controlled by the processor 40 in response to feedback from the pupil light detector 32, which can comprise, e.g., a CCD camera, or reticon detector array to monitor the size of the pupil, so that a predetermined pupil size can be maintained for the patient during the measurement. The locations of the pupil detection unit, including the components, beamsplitter 30, lens 33, and pupil light detector 32, can be in other appropriate locations along the optical path of the return light from the eye, including locations inside the optical relay unit 34.

As a further improvement to the accuracy of the refraction measurement, the system also monitors the first Purkinge image, an image formed by reflection at the anterior surface of the cornea of the light beam 26. The position of this image relative to the pupil boundary is an indication of gazing direction of the patient under examination. Unless the patient has strabismus in that eye, the relative position of the First Perkinge image is a well defined bright spot, and it is typically inside the pupil boundary. Therefore, in a preferred but non-limiting embodiment, the pupil detector 32 can also function as a patient gazing monitor. In this case, the relative position of the First Perkinge image to the pupil is determined by processing of the image data from the CCD camera, for example, using data filtering, contrast enhancement, and pupil boundary determination methods known in the art.

In various embodiments, software processing can be done in real time in a matter of a fraction of a second. The objective of this analysis is to determine whether the patient is looking at the target, or momentarily drifting off. The information is electromagnetically transmitted to the central control unit 40. If the patient is not looking at the target, the data set from the wavefront detector unit 38 is rejected, and preferably is not displayed or accumulated for data analysis.

As indicated above, the following comments are germane to implementation details of preferred, non-limiting embodiments. The light source 24 can be a diode laser that emits light at near infrared wavelengths. Moreover, the light detectors 32, 38 can be implemented by CCD arrays or linear reticon arrays or other detection devices. Further, the primary beamsplitter 20 can be coated to transmit visible light and to reflect infrared light. On the other hand, the laser beamsplitter 28 can be a polarization dependent reflector, in which case the laser light is polarized, and a quarter wave plate (not shown) is disposed in the beam path to the patient 15 such that the return beam is rotated ninety degrees (90°) upon double passing the quarter wave plate for facilitating passage thereof through the laser beamsplitter 28 toward the wavefront analyzer optics 36. Alternatively, the laser beamsplitter 28 can be plate coated for high transmission and low reflectivity at forty five degrees (45°) incident angle, such that only a small portion of the laser light is reflected into the eye, but a high percentage of the return light propagates through the laser beamsplitter 28.

Continuing with the implementation details of a preferred, non-limiting embodiment, the optical relay unit 34 can include two convex lenses F1 and F2 that together establish a telescope. The lenses F1, F2 are separated from each other by a distance equal to the sum of their focal lengths, with the focal plane of the first lens F1 being located at the front surface of the test lens 22, i.e., the surface facing the primary beamsplitter 20. In the case where the lens 22 is not between the patient's eye 15 and the primary beamsplitter 20 (e.g., wherein the test lens is between the primary beamsplitter and the target 18), the focal plane of the first lens F1 is preferably located at the front of the eye, i.e. the front of the cornea. The focal plane of the second lens F2 is located at the image plane of the wavefront analyzer optics 36. The purpose of the telescope structure of the relay unit 34 is to relay the wavefront at the exit surface of the test lens 22 to the image plane of the wavefront analyzer optics 36. Alternative relay optics can be used to achieve the same or different purpose.

With respect to the non-limiting details of the wavefront analyzer optics 36, the optics 36 can include an array of lenslets arranged as in a Shack-Hartmann wavefront sensor, an example of which can be found in page 37, "Customized Corneal Ablation The Quest for Super Vision" edited by MacRae, et. al. published by Slack Incorporated, 2001, incorporated herein by reference. Various Shack-Hartmann wavefront sensors and processors are available, for example, from commercial vendors such as Wavefront Sciences, in Albuquerque, NM, Zeiss/Humphrey Instruments, in Dublin, CA., or Bausch and Lomb, in Irvine, CA. More preferably, the optics 36 can include ruled reticles such as those disclosed in co-pending application U.S. patent application serial no. 10/014037, entitled "SYSTEM AND METHOD FOR WAVEFRONT MEASUREMENT", filed December 10, 2001, and U.S. patent application serial no. 10/314,906, entitled "SYSTEMS AND METHODS FOR WAVEFRONT MEASUREMENT", filed December 9, 2002, which use a self-imaging diffraction principle to detect the wavefront in the return beam.

Regardless of the type of wavefront analyzer optics 36 used, the processor 40 analyzes the profile of the wavefront of the light returned from the patient's eye, and quantifies the wavefront aberrations in two regimes: low order aberrations, including spherical refractive error, cylinder, and axis, and higher order aberrations, including coma, spherical aberrations and other higher order terms that can be described by Zernike polynomials. Quantitative data representing the patient's quality of vision are then graphically presented on the display device 42.

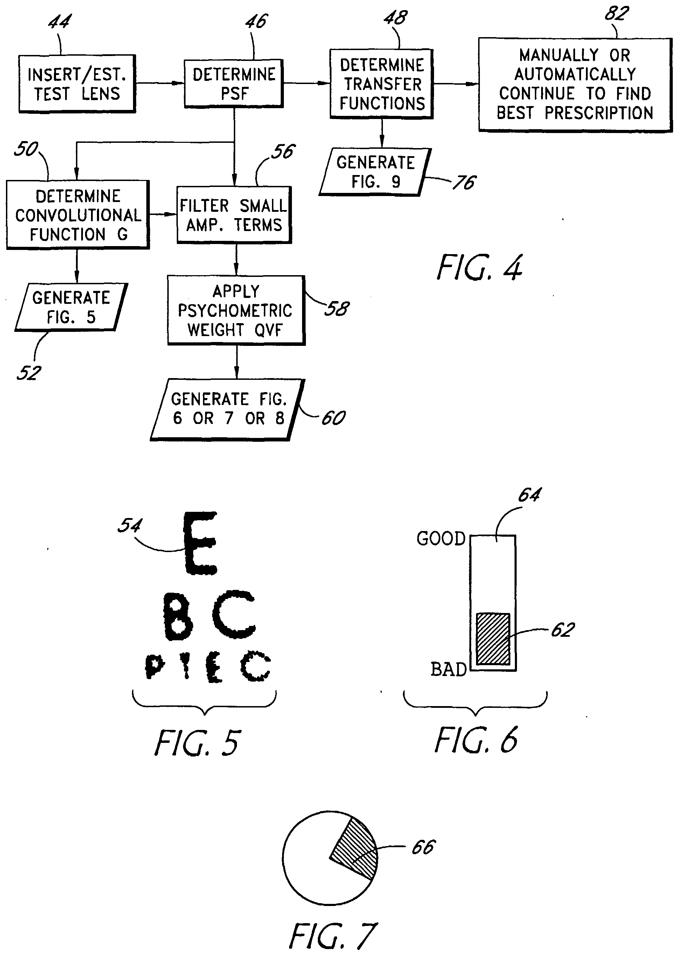

Now referring to Figure 4, an exemplary mode of operation of an apparatus such as depicted in Figure 3 can be seen. The patient 15 views the target 18 through the system 10, and in particular through the transparent window that is established by the primary beamsplitter 20. At block 44, the examiner initiates the vision test by inserting a selected test lens 22 in the line of sight of the patient, or by configuring a variable focal length lens 22 to have a predetermined focal length. Inserting means either manual positioning or positioning using a motor or other translation or rotation mechanism. In certain embodiments, the processor 40 can select a particular lens 22 and cause it to be automatically moved in the line of sight, in accordance with discussion below.

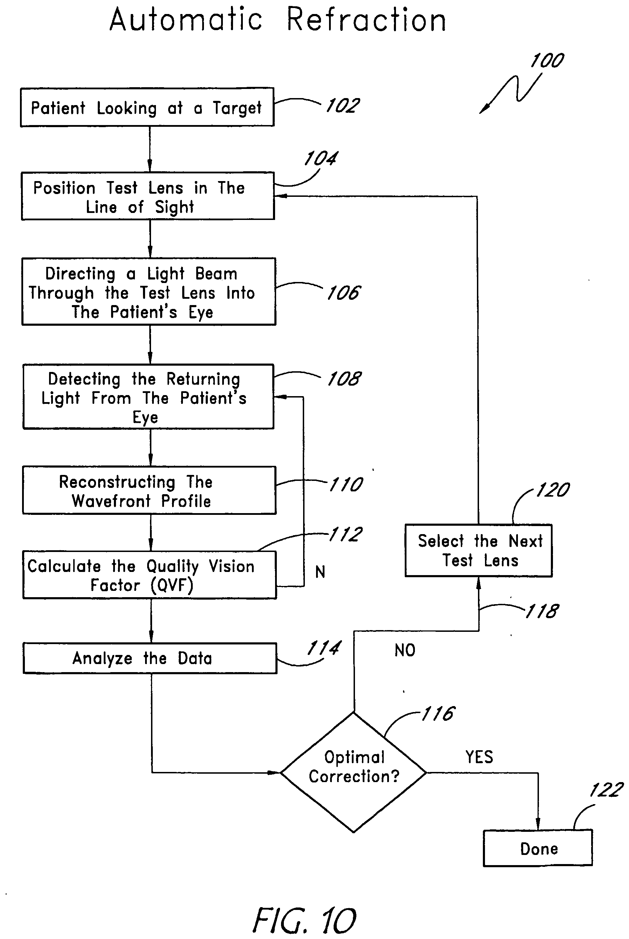

Various steps represented by blocks in Figure 4 involve calculations, and other types of activities that can be automated and at least partially implemented using for example computer logic. In particular, in accordance with processes and methods described and shown herein. These methods and processes include, but are not limited to, those depicted in at least some of the blocks in the flow chart of Figure 4 as well as the flow chart in Figure 10, which is described more fully below. Moreover, those skilled in the art will appreciate that the flow charts and description included herein illustrate the structures of logic elements, such as computer program code elements or electronic logic circuits. Manifestly, various embodiments include a machine component that renders the logic elements in a form that instructs a digital processing apparatus (that is, a computer, controller, processor, etc.) to perform a sequence of function steps corresponding to those shown.

In other words, the logic may be embodied by a computer program that is executed by the processor 40 as a series of computer- or control element-executable instructions. These instructions may reside, for example, in RAM or on a hard drive or optical drive, or the instructions may be stored on magnetic tape, electronic read-only memory, or other appropriate data storage device.

Proceeding to block 46 in Figure 4, the processor 40 determines the point spread function (PSF) that is derived from using, for instance, the terms of Zernike polynomials, which is in turn derived from the wavefront passing through the wavefront analyzer optics 36 and transformed into an electrical signal by the wavefront detector 38 when the wavefront data is acquired. The processor 40 may Fourier transform the signal from the wavefront detector 38 using the following equation:

$$PSF(x, y) = \left| FT(P(x, y)) \right|^2$$

wherein FT designates a Fourier Transform calculation and P(x, y) is the spatial distribution of the wavefront profile of light with the same phase (i.e., phase front) returned at the corneal plane.

Proceeding to block 48, if desired, an Optical Transfer Function (OTF) can be calculated from an inverse operation of Fourier Transform as follows:

$$OTF(f_x, f_y) = FT^{-1}(PSF(x, y)),$$

wherein $f_x$, $f_y$ are spatial frequencies in x and y directions, respectively, that are orthogonal to each other.

Moreover, a Modulation Transfer Function (MTF) can be determined as the amplitude of the OTF:

$$MTF(f_x, f_y) = \left| OTF(f_x, f_y) \right|.$$

The above functions are used to generate visual indications of the quality of vision that is afforded by the test lens 22 currently being viewed by the patient 15. For instance, once the PSF is determined at block 46, the logic can flow to block 50 to determine a convolution function G as follows:

$$G(X_{img}, Y_{img}) = \iint PSF(x - x_{img}, y - y_{img}) f(x_{img}, y_{img}) dxdy,$$

wherein $f(x_{img}, y_{img})$ is the test target 18 (Figure 3), i.e., an ideal image function, $x_{img}$-$x_{img}$ is the difference in the x-dimension between each point in the PSF and the corresponding ideal point in the ideal image, and $y$-$y_{img}$ is the difference in the y-dimension between each point in the PSF and the corresponding ideal point in the ideal image.

The convolutional function G can be used at output state 52 to generate an appropriately blurred image, point by point, of an ideal image as affected by the imperfection of the patient's eye in combination with the lens 22. For example, when the target 18 is a Snellen chart, the ideal image function can be the letter "E" or a series of other letters, e.g., of various physical sizes as conventionally used in the various lines in the Snellen chart. Figure 5 shows one such blurred image at 54, which can be presented on the display 42. Alternatively, the target can be a picture, and the convolved image $G(x_{img}, y_{img})$ of the picture is blurred point by point, according to the PSF, which represents an image of the target formed at the patient's retina.

Accordingly, the letters in the simulated blurred image have the same blurring as perceived by the patient 15. In this way, the examiner can visualize the clarity and sharpness of the image as a result of the lens 22 as it is perceived by the patient 15.

Alternatively or in addition to the image shown in Figure 5, the processor 40 can generate the displays shown in Figures 6-8 as follows. At block 56 the wavefront profile, as indicated by the above-mentioned linear combination of Zernike polynomials, is filtered to eliminate terms with coefficient below a threshold amplitude. Moving to block 58, a psychometric weighting factor "P" is inserted for each of the remaining Zernike terms as shown in the following. This weighting factor "P" represents the effect of the brain to discriminate objects despite certain types of ocular aberrations. For example, most people can discern a letter in a Snellen chart with a certain amount of defocus while the same amplitude of aberration in coma would not allow the same patient to discern that letter. To compensate for this, a Quality of Vision Factor (QVF) is determined as follows:

$$QVF = \exp^{-(\Sigma n P n Z n^2)}$$

wherein $P_n$ is the psychometric weight factor for the corresponding $n^{th}$ term of the Zernike polynomials, and $Z_n$ is the coefficient of the $n^{th}$ term of the Zernike polynomials in the PSF.

The psychometric weighting factors "$P_n$" can be determined by presenting a particular aberration to a normative group of people representing an average population distribution, for example, between about two hundred to several thousand people, depending on the accuracy level desired and the range of the scatter value of each type of aberration measurement. The patients are presented one at a time with particular types of aberrations selected from the Zernike polynomials, and then the patients are scored for the extent of success in discerning the target, for example, by the number of letters correctly read on the Snellen chart, or other standardized vision test chart. Other methods based on contrast level, standardized letter size, sine or square wave patterns can also be used. For example, success of patients in discerning graphics consisting of lines having various cycles per degree may be employed to test contrast sensitivity.

The optical element for introducing such aberration can be an aberration plate on which a selected type of aberration has been imprinted. For example, the aberration type can be a coma, or a trefoil, having a refractive index profile to produce the proper phase changes as specified by the corresponding Zernike polynomial.

The relative effect of each type of aberration is tabulated for the group and averaged to obtain statistically meaningful weight factors. For most practical purposes, Zernike coefficients for terms higher than the sixth order (term number twenty nine or higher) minimally contribute to the overall aberration profile of normal eyes. The method of producing various types of aberrations with desired amplitudes according to each of the Zernike terms is set forth in co-pending U.S. patent application serial no. 09/875,447 filed June 4, 2001 and entitled "WAVEFRONT ABERRATOR AND METHOD OF MANUFACTURING". In the event that no data for the psychometric weight factors are available, all weighting factors $P_n$ can be set to unity.

Once the QVF has been determined, one or more of the displays shown in Figures 6-8 can be generated and displayed as indicated in block 60. For example, as shown in Figure 6, a bar chart display can be generated to present an overall indication of the patient's quality of vision as afforded by the lens 22 under test. As shown, the indicator can be in the form of a graduated bar 62, the height of which is proportional to the QVF determined at block 60, with zero indicating poor vision and perfect vision being indicated by a bar extending from the bottom to the top 64 of the scale. If desired, the display scale can be a log scale, in which case the "best" is indicated by the bar 62 being at zero, and rising logarithmetrically with worse vision using a root-mean-square function of the QVF. Enhancements to the bar 62 such as color-coding can be used. For instance, the bar 62 can be colored red for poor vision and green for good vision, with other colors being used to indicate intermediate qualities of vision. In lieu of the bar chart of Figure 6, the QVF can be used to generate a pie chart as shown in Figure 7, with the size of a pie slice 66 relative to the entire circle being linearly or logarithmetrically proportional to the QVF.

The system 10 can substantially continuously measure the wavefront profile of the light beam returned from the patient's eye. Accordingly, in one preferred, non-limiting embodiment, a sequence of QVF measurements (e.g., twenty) for a single test lens 22 can be made in a second or two and grouped together.

As described earlier, the measurement accuracy can be improved by monitoring the gazing direction of the patient, and the computing device in block 40 can reject the data points acquired when the patient was not looking at the designated target. Furthermore, the computing device can also accumulate data and perform calculations for average values and standard deviation for selected subsets of measurement.

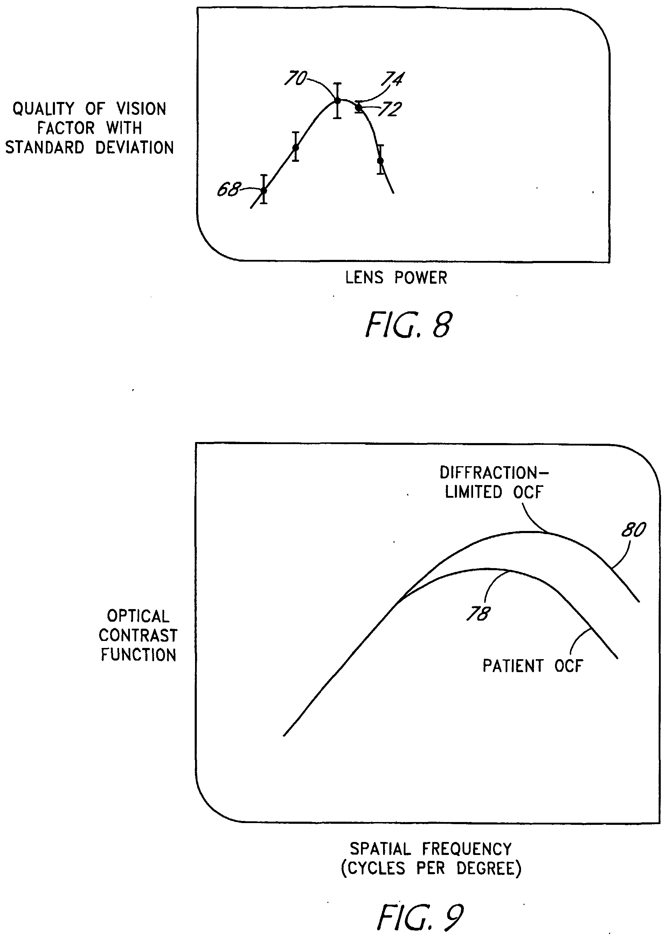

Figure 8 shows a resulting display. As can be appreciated from the exemplary embodiment shown, five lenses 22 have been tested and several QVF values obtained over a short period for each. Each group of QVF values is plotted as a respective vertical line 68 on a plot of QVF versus lens, with the length of each error bar line 68 representing the standard deviation of the measurements for that particular lens and the center of each line representing the mean QVF value. The prescription can be based not only on a high mean QVF value, as indicated at points 70 and 72, but also on a small standard deviation, as indicated by bar 74. That is, the lens corresponding to the bar 74 might be selected because its QVF values had a small standard deviation from each other and it had a high mean QVF value, even if not as high as the point 70. This recognizes that some patients prefer a lens power which may not necessarily provide the sharpest image but which does result in more comfort, since a patient does relatively little searching for the "better focus" using lenses that exhibit smaller standard deviations in the QVF. An example case is a patient who has not had a vision check for an extended period of time and requires correction of more than 1.5 diopters in cylinder. The patient may feel uncomfortable with the full correction as afforded by the sharpest image; rather the patient may prefer a smaller amount of correction which represents improvement in his vision, yet that does not cause dizziness or head strains. Therefore, embodiments of the present invention may provide objective data based on the subjective response of the patient to a test lens set 22 presented to the patient.

Determining the variation in the figure of merit preferably involves performing multiple measurements with consecutive measurements separated by a sufficiently long time interval. This time interval is preferably long enough to permit the eye to adjust its focus. Thus, the variation in data points should reflect adjustments made by the eye such as are present when the eye is straining to focus or accommodate. The time period between consecutive measurements is therefore preferably between about 0.1 to 5 seconds, however, values outside this range are also possible.

Accordingly, various test conditions may be presented to the patient under examination to investigate the subjective responses to such conditions. For example, as described above with reference to Figure 8, objective measurements are performed to determine the subjective response of the patient to different optical correction. By taking into account the subjective aspect of the patients vision experience, a prescription for correction of the refractive errors that is superior to that obtained with conventional objective measurement approaches is possible. Other conditions can be altered and the subjective response of the patient determined using the objective measurements described herein. Fogging and testing for the range of the accommodative ability are some other examples of how conditions that affect vision may be controlled and tested. In each of these cases, the apparatus and methods described herein advantageously can enables quantification of the subjective response of the patient by performing objective measurements, i.e., without any verbal communication with the patient.

As recognized herein, to determine a patient's ability to resolve patterns having varying levels of contrast, the patient can be presented with a series of standardized patterns of sine wave gratings of increasing spatial frequency frequencies, with the patient offering subjective responses. The resulting examination report can be a curve that depicts a cutoff contrast intensity at various spatial frequencies. The present disclosure can provide a quantitative evaluation of a patient's ability to handle contrast, which can be generated in addition to or in lieu of those displays discussed above. Such a display can be generated at output state 76 in Figure 4 and is shown in Figure 9, showing a curve 78 depicting actual patient optical contrast function (OCF) and a reference curve 80 depicting a diffraction-limited reference. To determine OCF, the following relation may be used:

$$OCF = MTF \times M_{lat.}$$

wherein MTF is the modulation transfer function determined at block 48 and $M_{lat}$ is a mathematical function accounting for the low frequency filtering of the neural system, the value of which linearly increases with spatial frequencies with a slope of unity in a log-log scale plot and reaches the maximum value of one at and above the spatial frequency of seven cycles per degree.

Accordingly, the OCF does not include the effect of the brain processing at frequencies higher than seven cycles per degree, but it does provide valuable information about the patient's ability to discern sine gratings of various spatial low frequencies based on a patient's optics.

The above process of measuring (and displaying) indications of the improvement in vision afforded by a particular test lens 22 to the patient 15 can be continued at block 82 until the "best" test lens is found. This can be done by the examiner manually swapping lenses 22 as, e.g., in a conventional phoropter. As mentioned above, in other embodiments, the positioning of test lenses 22 can be done automatically by the processor 40 controlling the moving mechanism "M", which can include a motor (or actuator) and coupling structure connecting the motor/actuator to one or more lenses 22. With this configuration, the mechanism follows an instruction from the processor 40 to insert the particular lens in the line of sight of the patient, as requested by the processor.

When done by the processor 40, the sequence of test lenses 22 to be used in an examination may be programmed into the processor 40 in accordance with examination strategy and routines known in the art. The starting lens can be selected based on the patient's current spectacle prescription or based on the wavefront measurement without any test lens 22 in the patient's line of sight. Consequently, in reconstructing the return beam wavefront without a test lens 22 being in the beam path, the processor 40 essentially models the uncorrected aberrations of the patient's eye in Zernike terms.

Recall that the second order Zernike terms represent defocus, astigmatism and axis information. Based on the patient's pupil size and the uncorrected wavefront error amplitudes, the processor 40 can determine the equivalent diopter power in sphere and cylinder and its axis, and select the appropriate lens 22 being used to start the examination. In selecting test lenses 22, the processor 40 can use the above-disclosed QVF values or other figures of merit in lieu of subjective responses from the patient, and can then execute the examination strategy as if it is performed with the subjective response from the patient.

The automatic refraction process is depicted in Figure 10, and generally designated 100. Process 100 begins with a first step 102 wherein the patient looks at a target. In step 104 a test lens is positioned between the target and the patient's eye, and in the line of sight of the patient. A light beam is directed through the test lens and into the patient's eye in step 106. As discussed earlier, the test lens may be positioned outside of the light beam, yet in the line of sight of the patient, positioned between the beam splitter 20 and the target 18 in Figure 3. In this configuration, the patient's vision is affected by the test lens as he is looking at the target, but the wavefront emerging from the patient's eye is directed to the wavefront sensor without passing through the test lens, and thus this wavefront contains both the low order and higher order aberrations of the patient's eye.

In step 108, the light returning from the patient's eye is detected. From this detected light, the wavefront profile may be reconstructed, as shown in step 110. From the reconstructed wavefront profile, the quality vision factor ("QVF") may be calculated in step 112. In order to improve the accuracy of the measurements of the patient's eye, "N" number of measurements are performed using the returning light. Accordingly, "N" wavefront measurements are taken, thereby yielding N wavefront profiles and corresponding QVF values. These successive measurements are taken by returning from step 112 to step 108, in which the returning light is again detected. Steps 108 through 112 are executed "N" times for the test lens. As described above, the "N" measurements are spaced apart in time by a sufficient amount to allow the patient to adjust their focus. For example, this time interval is preferably long enough to permit the muscles in the eye to readjust.

Once "N" measurements have been taken and the QVF for each measurement has been calculated, the QVF for that particular test lens is analyzed in step 114. The analysis of this data provides for a determination whether the correction with that particular lens is optimal. This decision is made in step 116, and if the correction is not optimal, a next test lens is selected in step 120, and the process returns to step 104 where the next test lens is positioned in the patient's line of sight. On the other hand, if the correction with that particular lens is optimal, then the process ends in step 122 resulting in the proper refractive correction having been identified. In other embodiments, the optimal lens may not be identified immediately after that particular lens is tested on the patient. Instead, the suitable lens may be selected after trying one or more additional test lens. Such is the case wherein a figure of merit analysis like that illustrated in Figure 8 is employed.

In a typical refraction process, a series of lenses in 1/4 diopters increments are used to determine the patient's optical correction. However, in various embodiments of the present invention, more or less than the typical number of lenses may be used, and the diopter increments can be in 1/8 instead of 1/4, depending upon the magnitude of correction necessary. Also, as shown in Figure 10, a number ("N") of measurements of the returning light are performed in steps 108 through 112 to calculate the QVF for the particular test lens. In some embodiments, for example, 5-20 separate measurements (i.e., "N" equals 5-20) are performed to provide an accurate measurement of the QVF. However, more or less measurements may be performed depending upon the particular wavefront sensor device used, and the magnitude of correction necessary.

The prescription can be determined by a person viewing the display, e.g., of Figure 8, or automatically by the processor 40 based on a high QVF value and low standard deviation, as follows: The examiner, or the processor 40 will examine the curve shown in Figure 8, which connects the average values of the QVF's for various lens sets presented to the patient. Certain embodiments may include the step of performing a best fit to the average values, using a polynomial of up to 4 orders, for example. The processor 40 searches for the maximum value, of the "peak" of the curve. This can be accomplished by monitoring the peak value of the curve, or the slope of the curve, e.g., from decreasing lens power, from right to left, in Figure 8. When the curve reaches its maximum value, the slope changes sign, and the maximum is at the zero slope value. Now, the processor 40 sends the peak value of the QVF and the corresponding lens power to the display or a printer.

Additionally, the processor 40 may also search for the minimum value among the standard deviation in the data set such as shown in Figure 8. A figure (not shown) similar to Figure 8 showing standard deviations versus lens power can be useful in determining the minimum value. Again curve fitting can be employed. The minimum value may be identified for example by detecting the sign change in the slope as described above for determining the maximum QVF value. Again, this minimum standard deviation value and the corresponding lens power are sent to the display device or a printer for record. For example, on the display or the printout the processor may indicate that lens power with the maximum QVF value provides for the sharpest image, while the lens power with the minimum standard deviation provides for the most comfortable prescription to the patient.

In certain embodiments both the average value of the figure of merit and its variation, e.g., the standard deviation, may be employed together to determine the suitable correction. The optical correction selected may for example be substantially equal to an amount between the optical correction yielding the least variation and the optical correction yielding the least aberration.

While Figure 3 illustrates a system 10 wherein the return beam from the lens 22 is detected and analyzed and, hence, the integrated effect on the wavefront introduced by the eye and lens 22 is measured, it is to be understood that as mentioned above the return beam from the eye can also be analyzed without passing through the lens 22. The test lens 22 may for example be located between the primary beamsplitter 20 and the target 18. In such an embodiment, the effect of the lens 22, which has a known deviation from spherical, can be accounted for by adding or subtracting the lens 22 effect as appropriate from the eye-only wavefront. To facilitate this, in some embodiments a sensor (not shown) can be provided that senses which lens 22 is moved into the patient's line of sight. The sensor sends a signal representative of the lens 22 (and, hence, of the optical contribution of the lens) to the processor 40.

In any case, it may now be appreciated that if desired, the examiner can use conventional tactics in the steps of selecting test lenses 22 as if the whole process were done using a conventional phoropter that requires subjective responses from the patient. For example, the examiner can use "fogging" in accordance with principles known in the art to form an artificial image before the retina of the patient to cause the patient to relax the above-mentioned accommodative power. However, as a result of the display capability of the system 10, the examiner can identify the test lens 22 with which the patient achieves good vision without accommodation, regardless of patient verbal cooperation or ability to judge and articulate which lens 22 is preferable.

Advantageously, embodiments described herein may be suitable for quantifying accommodation. For example, the variation in the vision figure of merit may be determined for a variety of conditions that may induce differing amounts of accommodation. These conditions may include, for instance, distances from the eye to the target. Additionally, these conditions may include different amounts of optical power in the case wherein optics are inserted in the optical path between the eye and the target. By varying these conditions, the eye may accommodate. This accommodation may be characterized, for example, by determining the average value of the N measurements and the variation in the figure of merit values with varying condition and its corresponding accommodation response of the patient. In one approach the examiner may observe the displays and continue to decrease the focusing power (or increase minus power) of the test lenses being used, thereby moving the image behind the patient's retina. For example, if the patient is under the age of 40 years old, the patient's accommodative power may be in the range of 1 to 5 diopters, with younger patients tending to have greater accommodative power. For young hyperopic patients, determining the onset of accommodation is typically an important task to avoid an over minus corrective power in prescribing spectacle lenses. Using a preferred method, the examiner may find both the accommodative onset and the range of accommodation. The examiner may start with a higher corrective power lens than what is optimal for the patient that causes the image of the target to fall in front of the retina. The approximate optimal power may be determined from an autorefractor measurement, or measured directly from the patient's spectacle lenses. The starting power for the test lens is then preferably chosen to be 1 to 3 diopters higher than the approximate optimal power. The quality of vision and the figure of merit value is below the optimal value at this point. Then the examiner may decrease the corrective power of the test lens, thereby bringing the target into sharper focus, and an improved figure of merit of vision which is also shown by the increase of the average value of the figure of merit. This continues until a peak average value of the figure of merit is reached. This peak value is noted as the actual optimal refractive correction for the patient. So far, the examiner has performed a process that in many respects is similar to a traditional fogging, except that the entire procedure is done using a objective method and no verbal communication is needed. For older patients who have no accommodative power, any further decrease of the refractive power of the test lens would cause the figure of merit value to drop. However, for the young patients, accommodation would be applied to cancel the over minus effect of the test lens, so that the patient's focusing effort results in a sharp image of the target. The average value of the figure of merit does not drop appreciably at this point, but only slightly and leveling to form a plateau. Until the over minus power exceeds the accommodative range of the patient, the figure of merit value curve starts to drop. The width of the plateau is an indication of the patient's range of accommodative power. One aspect of this method is that while the patient may be able to see the target with reasonable sharpness using his accommodation, the extra efforts to drive the accommodation causes the focusing power of the eye to be unstable, which is manifested in the variation of the figure of merit value, or more specifically from the standard deviation calculation based on the number of repeated measurements. The error bars representing the standard deviation if plotted over the average value curve as a function of the diopter power of the test lenses, similar to the one shown in figure 8 would typically show larger standard deviations with more efforts to accomplish focusing when looking through the over minus power lenses (curve not shown). For a young hyperopic patient, who has not had his eye properly corrected, he may be conditioned to accommodate and often times the patient actually prefers to accommodate while looking at distant objects. In this case, the standard deviation error bar may be smaller than at the zero accommodation point where the curve first reaches its maximum value. If in fact this is the case, a prescription may, for example, be chosen to be a mid point between the zero accommodation point and the point with the minimum standard deviation. This example illustrates an aspect of this method in that the figure of merit data together with its standard deviations over N measurements, N may be in the range of, e.g., 3 to 10, are manifesting the subjective response of the patient as he reacts to various test lenses or other stimuli. Therefore, the aspect of using the average and the deviation of a figure of merit of vision has the ability of detecting subjective responses by studying the variation of the figure of merit over repeated measurements of the same test lens while the entire measurement is performed in an objective manner. The examiner can thereby determine the range of accommodation of the patient based on the objective measurements provided by the apparatus 10 and also provide a prescription to reduce accommodation or hyperopia errors.

As described above, in various embodiments, repetitive measurements used in determining optical correction, accommodation, or other vision properties are spaced apart over time intervals that are sufficiently long to allow the person under examination to adjust the focus of their eye. Consequently, the information gathered by the system represents the physical response of the person to the various conditions to which they are exposed. Thus, by allowing the person time to adjust to the various test conditions, and measuring the quality of vision responding to such conditions repeatedly, objective measurements can be obtained of the subjective responses of the patient without communicating with the person verbally, which is required when performing a manifest refraction using a conventional phoropter.

Specific examples of conditions that may be modified to characterize a person vision may include but are not limited to variations in test optics that are positioned in the line of sight as well as changes in the target distance. The target distance can be modified by moving the physical target. In addition, the apparent distance of the target can be changed by manipulating the beam of light from the target to the eye.

The apparent distance of the target can be controlled by altering the beam of light from the target to the eye. The patient's eye collects a portion of the light emanating from the target. The divergence angle of these collected rays will depend on the location of the target. For example, as is well known, light from a point object at the infinity is collimated (i.e., the divergence is approximately zero). An object at a closer distance will have a larger angle of divergence. As a result, the eye will perceive the target to be at different locations based on the divergence of the rays of light entering the patient's eye. Thus, by adjusting the divergence properties of the rays of light or the associated wavefronts, the apparent distance of the target seen by the patient may be controlled. This may be accomplished for example by adjusting the optics. Such adjustments to the viewing conditions to which a patient is exposed may help the examiner characterize the patients vision and provide suitable correction.

Once the appropriate lens 22 has been identified, the examiner may indicate this decision by pressing a "finish" button (not shown), and a printout of the examination result can be output using a printer or similar device (not shown) that is connected to the processor 40. The processor 40 may also automatically transmit the prescription via modem, internet or other appropriate communication medium to a remote location for lens manufacturing. A prescription to correct low order aberrations including sphere, cylinder and axis, can be used for prescribing conventional ophthalmic lenses, or a "supervision" prescription to correct higher or even all orders of aberrations can be used to provide improved vision lenses, such as are described in co-pending U.S. patent application serial no. 10/044,304, filed October 25, 2001 and entitled "EYEGLASS MANUFACTURING METHOD USING VARIABLE INDEX LAYER".

Accordingly, the apparatus and methods described herein advantageously enable quantification of the subjective response of the patient by performing objective measurements, i.e., without relying on verbal communication with the patient regarding the quality of their vision. Namely, objective measurements can be performed that determine the subjective response of the patient to different optical correction. By taking into account the subjective aspect of the patient's vision, a prescription for correction of the refractive errors can be obtained that is superior to that determined by conventional objective measurement approaches.

Those skilled in the art will appreciate that the methods and designs described above have additional applications and that the relevant applications are not limited to those specifically recited above. Also, the present invention may be embodied in other specific forms without departing from the scope of the invention defined by the appended claims.

**Claims**

1. An apparatus for automatically determining the patient's quality of vision, said apparatus comprising:

   (a) test optics for testing said vision, said test optics being variable to provide different amounts of optical correction;
   (b) a light source (24) and associated optics (20, 28) for directing light into an eye (15) of the patient, said light reflecting from tissue in said eye, said reflected light comprising a plurality of wavefronts that propagate through an ocular lens and cornea in said eye;
   (c) a wavefront sensor (38) including one or more optical detectors for measuring said wavefronts emanating from the patient's eye, said wavefront sensor (38) having an electrical output representative of said wavefront;
   (d) an electrical processor (40) configured to receive said electrical output from said wavefront sensor (38) and
   (e) a mechanical actuator (44) electrically connected to said processor, said mechanical actuator (44) adjusting the test optics so as to vary the amounts of optical correction
   (f) a computer readable medium having a program of instructions stored thereon for causing said electrical processor (40) to execute method steps for determining the quality of said patient's vision, comprising calculating a value of a figure of merit indicative of the patient's quality of vision, said value being derived at least in part from a measurement of one of the wavefronts from the patient's eye,

   **characterized in that**
   said figure of merit indicative of quality of a

   person's vision comprises a Quality of Vision Factor (QVF) defined as $QVF = \exp(-\sum P_n Z_n^2)$, wherein $Z_n$ corresponds to coefficients for Zernike polynomials and $P_n$ correspond to psychometric weight factors.

2. The apparatus of Claim 1, wherein the executed method steps for determining the quality of said patient's vision further comprises: (I) retrieving another wavefront measurement from the wavefront sensor; (II) calculating a value of a figure of merit indicative of the patient's quality of vision, said value being derived at least in part from a measurement of one of the wavefronts from the patient's eye; (III) repeating steps (I) and (II) N times; (IV) calculating the average and variation of the figure of merit values and storing the average and variation as a data set for a given optical correction; (V) instructing the actuator (44) to alter the test optics to provide a different optical correction; (VI) repeating steps (I) through (V) until a predetermined end point has been reached; and (VII) selecting the optical correction that yields (i) the maximum average value of the figure of merit, (ii) the minimum variation of the figure of merit, or (iii) a value between those of (i) and (ii).

3. The apparatus of Claim 1, further comprising a display (42) electrically connected to said electrical processor (40) and configured to receive input from said electrical processor (40) such that said electrical processor can output results of said measurements to said display (42).

4. The apparatus of Claim 3, wherein said electrical processor (40) and display (42) are configured to provide a graphical representation of a target (18) image as seen by the patient, said graphical representation generated by a convolution of said target (18) image with a function derived from said wavefront measurements, said image being distorted an amount indicative of distortion in the patient's vision, or are configured to provide information selected from the group consisting of (1) a numerical and/or graphic representative of the effectiveness of application of

different amount of optical power; and (2) a numerical and/or graphic display of the contrast function of the patient's vision.

5. The apparatus of Claim 1, wherein said electrical processor (40) includes electronic circuitry configured to select a prescription suitable for correction and an output for outputting said prescription.

6. The apparatus of Claim 1, wherein said test optics comprise a plurality of test lens (22) that can be separately introduced into an optical path between the patient's eye (15) and a target (18) to adjust the patient's vision.

7. A computer readable medium having a program of instructions stored thereon for causing the electrical processor of the apparatus of claim 1 to execute method steps for identifying optical correction for a person's eye based on wavefront measurements of light reflected from said person's eye, comprising: (a) calculating a value of a figure of merit indicative of the person's quality of vision, said value being derived at least in part from a measurement of a wavefront from said person's eye; (b) retrieving another wavefront measurement; (c) repeating steps (a) and (b) N times ; (d) calculating the average and variation of the figure of merit values and storing the average and variation as a data set for a given optical correction; (e) repeating steps (a) through (d) for different optical correction; and (f) selecting the optical correction that yields (i) the maximum average value of the figure of merit, (ii) the minimum variation of the figure of merit, or (iii) a value between those of (i) and (ii),
**<u>characterized in that</u>** said figure of merit indicative of quality of a person's vision comprises a Quality of Vision Factor (QVF) defined as $QVF = \exp(-\sum P_n Z_n^2)$ , wherein $Z_n$ corresponds to coefficients for Zernike polynomials and $P_n$ correspond to psychometric weight factors.

**Patentansprüche**

1. Vorrichtung zum automatischen Bestimmen der Visusqualität eines Patienten, wobei die Vorrichtung Folgendes umfasst:

(a) eine Testoptik zum Testen des Visus, wobei die Testoptik variabel ist, um unterschiedliche Ausmaße einer optischen Korrektur vorzusehen;
(b) eine Lichtquelle (24) und zugehörige Optik (20, 28) zum Lenken von Licht in ein Auge (15) des Patienten, wobei das Licht vom Gewebe im Auge reflektiert wird, wobei das reflektierte Licht mehrere Wellenfronten umfasst, die sich durch eine Augenlinse und Hornhaut im Auge ausbreiten;
(c) einen Wellenfrontsensor (38), der einen oder mehrere optische(n) Detektor(en) zum Messen der Wellenfronten enthält, die aus dem Auge des Patienten austreten, wobei der Wellenfrontsensor (38) einen elektrischen Ausgang hat, der für die Wellenfront repräsentativ ist;
(d) einen elektrischen Prozessor (40), der zum Empfangen des elektrischen Ausgangs vom Wellenfrontsensor (38) konfiguriert ist, und
(e) ein mechanisches Stellglied (44), das elektrisch mit dem Prozessor verbunden ist, wobei das mechanische Stellglied (44) die Testoptik so einstellt, dass das Ausmaß einer optischen Korrektur variiert wird,
(f) ein computerlesbares Medium, auf dem ein Programm von Anweisungen gespeichert ist, die den elektrischen Prozessor (40) veranlassen, Verfahrensschritte zum Bestimmen der Visusqualität des Patienten auszuführen, umfassend ein Errechnen eines Wertes einer Leistungszahl, die die Visusqualität des Patienten anzeigt, wobei der Wert zumindest teilweise aus einer Messung einer der Wellenfronten aus dem Auge des Patienten abgeleitet ist,

**dadurch gekennzeichnet, dass**
die Leistungszahl, die eine Visusqualität einer Person anzeigt, einen Quality of Vision Factor (QVF, Visusqualitätsfaktor) umfasst, der als

$$QVF = exp\left(- \sum P_n Z_n^2\right)$$ definiert ist, wobei $Z_n$ Koeffizienten für Zernike-Polynome entspricht und $P_n$ psychometrischen Gewichtungsfaktoren entspricht.

2. Vorrichtung nach Anspruch 1, wobei die ausgeführten Verfahrensschritte zum Bestimmen der Visusqualität des Patienten ferner umfassen: (I) Abrufen einer weiteren Wellenfrontmessung vom Wellenfrontsensor; (II) Errechnen eines Wertes einer Leistungszahl, die die Visusqualität des Patienten anzeigt, wobei der Wert zumindest teilweise aus einer Messung einer der Wellenfronten aus dem Auge des Patienten abgeleitet ist; (III) Wiederholen der Schritte (I) und (II) N Mal; (IV) Errechnen des Durchschnitts und einer Variation der Werte der Leistungszahl und Speichern des Durchschnitts und der Variation als Datensatz für eine bestimmte optische Korrektur; (V) Anweisen des Stellglieds (44), die Testoptik zu ändern, um eine andere optische Korrektur vorzusehen; (VI) Wiederholen der Schritte (I) bis (V), bis ein vorgegebener Endpunkt erreicht ist; und (VII) Auswählen der optischen Korrektur, die (i) den maximalen Durchschnittswert der Leistungszahl, (ii) die minimale Variation der Leistungszahl oder (iii) einen Wert zwischen jenen von (i) und (ii) ergibt.

3. Vorrichtung nach Anspruch 1, ferner umfassend eine Anzeige (42), die elektrisch mit dem elektrischen Prozessor (40) verbunden und konfiguriert ist, einen Eingang von dem elektrischen Prozessor (40) zu empfangen, sodass der elektrische Prozessor Ergebnisse der Messungen an die Anzeige (42) ausgeben kann.

4. Vorrichtung nach Anspruch 3, wobei der elektrische Prozessor (40) und die Anzeige (42) konfiguriert sind, eine grafische Darstellung eines Bildes eines Ziels (18), wie vom Patienten gesehen, vorzusehen, wobei die grafische Darstellung durch eine Konvolution des Bildes des Ziels (18) mit einer Funktion generiert wird, die aus den Wellenfrontmessungen abgeleitet ist, wobei das Bild in einem Ausmaß verzerrt ist, das eine Verzerrung im Visus des Patienten anzeigt, oder konfiguriert sind, Informationen vorzusehen, die aus der Gruppe bestehend aus (1) einer numerischen und/oder grafischen Darstellung der Wirksamkeit der Anwendung unterschiedlicher Ausmaße einer optischen Leistung; und (2) einer numerischen und/oder grafischen Anzeige der Kontrastfunktion des Visus des Patienten ausgewählt sind.

5. Vorrichtung nach Anspruch 1, wobei der elektrische Prozessor (40) einen elektronischen Schaltkreis, der konfiguriert ist, eine Verschreibung, die zur Korrektur geeignet ist, auszuwählen, und einen Ausgang zur Ausgabe der Verschreibung enthält.

6. Vorrichtung nach Anspruch 1, wobei die Testoptik mehrere Testlinsen (22) umfasst, die getrennt in einen optischen Weg zwischen dem Auge (15) des Patienten und einem Ziel (18) eingesetzt werden können, um den Visus des Patienten einzustellen.

7. Computerlesbares Medium, auf dem ein Programm von Anweisungen gespeichert ist, die den elektrischen Prozessor der Vorrichtung nach Anspruch 1 veranlassen, Verfahrensschritte zum Ermitteln einer optischen Korrektur für ein Auge einer Person basierend auf Wellenfrontmessungen von Licht, das vom Auge der Person reflektiert wird, auszuführen, umfassend: (a) Errechnen eines Werts einer Leistungszahl, die die Visusqualität der Person anzeigt, wobei der Wert zumindest teilweise aus einer Messung einer Wellenfront aus dem Auge der Person abgeleitet ist; (b) Abrufen einer weiteren Wellenfrontmessung; (c) Wiederholen der Schritte (a) und (b) N Mal; (d) Errechnen des Durchschnitts und einer Variation der Werte der Leistungszahlen und Speichern des Durchschnitts und der Variation als Datensatz für eine bestimmte optische Korrektur; (e) Wiederholen der Schritte (a) bis (d) für eine andere optische Korrektur; und (f) Auswählen der optischen Korrektur, die (i) den maximalen Durchschnittswert der Leistungszahl, (ii) die minimale Variation der Leistungszahl oder (iii) einen Wert zwischen jenen von (i) und (ii) ergibt, **dadurch gekennzeichnet, dass** die Leistungszahl, die eine Visusqualität einer Person anzeigt, einen Quality of Vision Factor (QVF, Visusqualitätsfaktor) umfasst, der als $QVF = exp\left(-\sum P_n Z_n^2\right)$ definiert ist, wobei $Z_n$ Koeffizienten für Zernike-Polynome entspricht und $P_n$ psychometrischen Gewichtungsfaktoren entspricht.

**Revendications**

1. Appareil de détermination automatique de la qualité de vision du patient, ledit appareil comprenant :

(a) des optiques de test pour tester ladite vision, lesdits optiques de test étant variables pour fournir différentes quantités de correction optique ;
(b) une source de lumière (24) et des optiques associées (20, 28) pour diriger une lumière dans un oeil (15) du patient, ladite lumière étant réfléchie par le tissu dans ledit oeil, ladite lumière réfléchie comprenant une pluralité de fronts d'onde qui se propagent à travers une lentille oculaire et la cornée dans ledit oeil ;
(c) un capteur de front d'onde (38) comportant un ou plusieurs détecteurs optiques pour mesurer lesdits fronts d'onde émanant de l'oeil du patient, ledit capteur de front d'onde (38) ayant une sortie électrique représentative dudit front d'onde ;

(d) un processeur électrique (40) configuré pour recevoir ladite sortie électrique dudit capteur de front d'onde (38) et

(e) un actionneur mécanique (44) connecté électriquement audit processeur, ledit actionneur mécanique (44) ajustant les optiques de test de manière à faire varier les quantités de correction optique ;

(f) un support lisible par ordinateur sur lequel est mémorisé un programme d'instructions pour amener le processeur électrique (40) à exécuter des étapes de procédé de détermination de la qualité de vision dudit patient, comprenant le calcul d'une valeur d'un facteur de mérite indicatif de la qualité de vision du patient, ladite valeur étant dérivée au moins en partie d'une mesure de l'un des fronts d'onde provenant de l'oeil du patient,

**caractérisé en ce que**
ledit facteur de mérite indicatif de la qualité de vision d'une personne comprend un facteur de qualité de vision (QVF) défini par $QVF = \exp(-\sum P_n Z_n^2)$, $Z_n$ correspondant à des coefficients de polynômes de Zernike et $P_n$ correspondant à des facteurs de pondération psychométriques.

2. Appareil selon la revendication 1 dans lequel les étapes de procédé exécutées pour déterminer la qualité de la vision dudit patient comprennent en outre : (I) le recouvrement d'une autre mesure de front d'onde à partir du capteur de front d'onde ; (II) le calcul d'une valeur d'un facteur de mérite indicatif de la qualité de vision du patient, ladite valeur étant dérivée au moins en partie d'une mesure de l'un des fronts d'onde provenant de l'oeil du patient ; (III) la répétition des étapes (I) et (II) N fois ; (IV) le calcul de la moyenne et de la variation des valeurs de facteur de mérite et la mémorisation de la moyenne et de la variation sous forme d'ensemble de données pour une correction optique donnée ; (V) l'instruction à l'actionneur (44) de modifier les optiques de test pour fournir une correction optique différente ; (VI) la répétition des étapes (I) à (V) jusqu'à ce qu'un point final prédéterminé soit atteint ; et (VII) la sélection de la correction optique qui produit (i) la valeur moyenne maximum du facteur de mérite, (ii) la variation minimum du facteur de mérite, ou (iii) une valeur entre celles de (i) et (ii).

3. Appareil selon la revendication 1, comprenant en outre un afficheur (42) connecté électriquement audit processeur électrique (40) et configuré pour recevoir une entrée dudit processeur électrique (40) de telle sorte que ledit processeur électrique puisse produire les résultats desdites mesures sur ledit afficheur (42).

4. Appareil selon la revendication 3, dans lequel ledit processeur électrique (40) et ledit afficheur (42) sont configurés pour fournir une représentation graphique d'une image cible (18) vue par le patient, ladite représentation graphique étant générée par une convolution de ladite image cible (18) avec une fonction dérivée desdites mesures de fronts d'onde, ladite image étant déformée par une quantité indicative d'une distorsion de la vision du patient, ou sont configurés pour fournir des informations sélectionnées dans le groupe consistant en (1) une représentation numérique et/ou graphique de l'efficacité d'application d'une quantité différente de puissance optique ; et (2) un affichage numérique et/ou graphique de la fonction de contraste de la vision du patient.

5. Appareil selon la revendication 1, dans lequel ledit processeur électrique (40) comporte des circuits électroniques configurés pour sélectionner une prescription de correction convenable et une sortie pour produire en sortie ladite prescription.

6. Appareil selon la revendication 1, dans lequel lesdits optiques de test comprennent une pluralité de lentilles de test (22) qui peuvent être introduites séparément dans une trajectoire optique entre l'oeil (15) du patient et une cible (18) afin d'ajuster la vision du patient.

7. Support lisible par ordinateur sur lequel est mémorisé un programme d'instructions pour amener le processeur électrique de l'appareil de la revendication 1 à exécuter des étapes de procédé pour identifier une correction optique d'un oeil d'une personne en fonction de mesures de fronts d'onde de lumière réfléchis par l'oeil de ladite personne, comprenant : (a) le calcul d'une valeur de facteur de mérite indicatif de la qualité de vision du patient, ladite valeur étant dérivée au moins en partie d'une mesure d'un front d'onde provenant de l'oeil de ladite personne ; (b) le recouvrement d'une autre mesure de front d'onde ; (c) la répétition des étapes (a) et (b) N fois ; (d) le calcul de la moyenne et de la variation des valeurs de facteur de mérite et la mémorisation de la moyenne et de la variation sous forme d'ensemble de données pour une correction optique donnée ; (e) la répétition des étapes (a) à (d) pour une correction optique différente ; et (f) la sélection de la correction optique qui produit (i) la valeur moyenne maximum du facteur de mérite, (ii) la variation minimum du facteur de mérite, ou (iii) une valeur entre celles de (i) et (ii), **caractérisé en ce que** ledit facteur de mérite indicatif de la qualité de vision d'une personne comprend un facteur de qualité de vision (QVF) défini par $QVF = \exp\left(-\sum P_n Z_n^2\right)$, $Z_n$ correspondant à des coefficients de polynômes de Zernike et $P_n$ correspondant à des facteurs de pondération psychométriques.

*FIG. 2*

*FIG. 1*

FIG. 3

FIG. 4

FIG. 5

FIG. 6

FIG. 7

QUALITY OF VISION
FACTOR WITH
STANDARD DEVIATION

LENS POWER

FIG. 8

OPTICAL
CONTRAST
FUNCTION

SPATIAL FREQUENCY
(CYCLES PER DEGREE)

FIG. 9

# Automatic Refraction

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1153570 A1
- US 01403701 A
- US 31490602 A
- US 87544701 A
- US 04430401 A

### Non-patent literature cited in the description

- **LIANG J. et al.** Objective measurement of wave aberrations of the human eye with the use of a Hartmann-Shack wave-front sensor. *JOURNAL OF THE OPTICAL SOCIETY OF AMERICA,* July 1994, vol. 11 (7), 1949-1957

- **MACRAE.** Customized Corneal Ablation The Quest for Super Vision. Slack Incorporated, 2001, 37